# FASCICULE DE BREVET EUROPEEN

(11) **EP 2 588 075 B1**
(45) Date de publication et mention de la délivrance du brevet: **27.12.2017**
(21) Numéro de dépôt: 11744031.3
(22) Date de dépôt: 28.06.2011
(51) Int. Cl.: A61K 8/64, A61K 8/97, A61Q 5/00

(54) **UTILISATION D'UN EXTRAIT PEPTIDIQUE DE POIS EN TANT QU'AGENT ACTIF ANTIOXYDANT DANS UNE COMPOSITION COSMETIQUE**
VERWENDUNG VON EINEM PEPTIDEXTRAKT VON ERBSEN ALS ANTIOXIDANT IN EINER KOSMETISCHEN ZUSAMMENSETZUNG
USE OF A PEPTIDIC EXTRACT OF PEA AS AND ANTIOXIDANT IN A COSMETIC COMPOSITION

(30) Priorité: 01.07.2010 FR 1002788
(43) Date de publication de la demande: 08.05.2013
(73) Titulaire: ISP Investments Inc., Wilmington, DE 19805 (US)
(72) Inventeur: DAL FARA, Claude, 12446 Kherkonston New York (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2011/000374
(87) Numéro de publication internationale: WO 2012/001246

(56) Documents cités:
- EP-A1- 1 133 973
- WO-A1-2010/119191
- WO-A1-2010/122244
- WO-A2-2008/015341
- FR-A1- 2 944 795
- JP-A- 2007 302 615
- JP-A- 2009 143 869
- GOTTSCHALCK T E ET AL: "International Cosmetic Ingredient Dictionary and Handbook, passage", INTERNATIONAL COSMETIC INGREDIENT DICTIONARY AND HANDBOOK, XX, XX, vol. 2, 1 janvier 2004 (2004-01-01), pages 1394-1395, XP002428249,

## Description

La présente invention se situe dans le domaine des compositions cosmétique et pharmaceutique appliquées aux cheveux. La présente invention concerne l'utilisation d'un hydrolysat peptidique de pois en tant qu'agent actif antioxydant, ainsi que plusieurs procédés de traitement non thérapeutique utilisant une composition comprenant ledit extrait.

Les cheveux sont des annexes kératiniques au même titre que les poils, les cils, les sourcils ou encore les ongles. Ils ont un rôle physiologique de protection du cuir chevelu, mais surtout un rôle social et ce depuis longtemps dans l'histoire de l'homme. Les cheveux peuvent être de différentes natures, longs, courts, raides, bouclés....mais ils obéissent tous à la loi du cycle. En effet, les 100 000 à 150 000 follicules pileux qui forment une chevelure « normale » se renouvellent tous de façon cyclique, asynchrone et stochastique à partir d'un réservoir de cellules souches adultes folliculaires.

Le follicule pileux est une annexe cutanée autonome avec son propre contrôle hormonal, son propre cycle, une structure complexe et stable (Bernard B. A. ; Médecine/Sciences 2006 ; 22 : 138-43). Le cycle du cheveu se décortique en 3 phases : les phases anagène, catagène et télogène. La phase anagène, ou phase de croissance du cheveu, dure entre 3 et 7 ans (variable selon l'âge, le sexe et la zone du cuir chevelu). Cette phase est suivie par une phase de repos appelée catagène et dure environ 3 semaines. Alors que des processus d'apoptose ont lieu durant cette phase catagène, entrainant ainsi la chute du cheveu, la phase suivante appelée télogène va permettre à un nouveau bulbe de se former à partir d'un germe pileux qui va initier le cycle suivant (Arouete J., J. Med. Esth. Et Chir. Derm., Sept. 2005, 119, 165-167). Cette phase télogène durera, quant à elle, environ 3 mois. C'est ainsi que, sur une chevelure « normale », environ 85 % des follicules sont en phase de croissance, 2 % en phase de repos, et un peu plus de 10 % en phase de chute.

On évoque souvent le vieillissement de la peau, les agressions que subit la peau, mais les fibres kératiniques ne sont pas en reste. En effet, l'ensemble des fibres kératiniques, et les cheveux en particulier, subissent un grand nombre d'agressions de la part de l'environnement, comme les rayonnements UV, les agressions de type chimiques dues aux traitement infligés aux cheveux comme les colorations, permanentes, les agressions par la chaleur des brushings etc. En effet, toutes ces agressions ont un impact important sur les cellules qui composent le follicule pileux. Lesdites cellules subissent des phénomènes d'oxydation et de vieillissement prématuré dus aux radicaux libres. La formation de ces radicaux libres va entrainer des dommages au niveau de la cellule, ces dommages pouvant mener à la mort cellulaire encore appelée apoptose. Par exemple, il est connu que le peroxyde d'hydrogène inhibe la synthèse de pigments colorés, dont la mélanine, et est responsable du blanchissement des cheveux : avec l'âge, le taux d'enzymes dissociant le péroxyde d'hydrogène en oxygène et en eau diminue et ce dernier s'attaque à l'enzyme tyrosinase responsable de la synthèse de la mélanine. Le phénomène d'apoptose au niveau des cellules du follicule pileux se traduit par l'apparition de vacuoles au niveau des cellules qui forment la gaine externe du follicule pileux, l'augmentation de certains marqueurs comme la caspase-3 ou encore la catalase etc. Ainsi, de nombreuses molécules sont actuellement utilisées afin de limiter la formation de ces radicaux libres ; ces molécules sont alors dites antioxydantes. Il s'agit par exemple, des Vitamines E, A, B6, B12, B5, C, de la cystine, de l'acide folique, du sélénium, du zinc, du fer, du cuivre, du manganèse, des acides gras de la familles des omégas 3 et 6, de l'acide alpha-lipoïque etc.

Mais il existe toujours un besoin, notamment en cosmétique, de développer de nouveaux traitements ayant une activité antioxydante et permettant de limiter l'impact des agressions extérieures sur les cheveux.

C'est ainsi que la Demanderesse a mis en évidence l'effet antioxydant d'un extrait peptidique de pois sur les cheveux. Jusqu'alors, des hydrolysats de pois étaient utilisés en tant qu'agent pigmentant (FR 2 904 556), ou encore en tant qu'agent pour desquamer la couche cornée (JP09025225). Une demande de brevet a également été déposée concernant un hydrolysat peptidique de pois enrichi en peptides bioactifs de séquence Ala-Gly-Glu-Leu-Ser afin de protéger la peau et de renforcer la fonction barrière. Mais l'utilisation d'un extrait peptidique de pois, non enrichi en peptides bioactifs, n'a jamais été décrite en tant qu'agent antioxydant pour les cheveux.

Ainsi, le premier objet de la présente invention se rapporte à l'utilisation d'un hydrolysat peptidique de pois en tant qu'agent actif antioxydant dans une composition cosmétique destinée au soin des cheveux.

Un autre objet de la présente invention concerne, d'une part, un procédé de traitement non thérapeutique destiné à protéger les cheveux des stress oxydatifs et, d'autre part, protéger les cheveux des agressions extérieures.

La présente invention concerne donc l'utilisation d'un hydrolysat peptidique de pois en tant qu'agent actif antioxydant dans une composition cosmétique destinée au soin des cheveux.

On entend par « hydrolysat peptidique », un extrait comprenant un mélange de composés majoritairement représentés par des peptides ou des oligopeptides. Selon l'invention, on utilisera indifféremment les termes « hydrolysat peptidique », « extrait », « extrait solubilisé » ou « agent actif ».

On entend par « cheveux », l'ensemble des fibres kératiniques humaines et plus précisément les cheveux, les sourcils, les cils, les poils de barbe, de moustache, les poils pubiens et les ongles. Plus spécialement, l'invention s'applique aux cheveux et/ou aux cils humains.

La Demanderesse a découvert qu'un extrait peptidique de pois présente une activité certaine sur les follicules pileux des cheveux et, en particulier, avait une action en tant qu'agent protecteur contre plusieurs types d'agressions extérieures grâce à son action antioxydante. En effet, la Demanderesse a démontré à l'aide de tests *in vitro,* que l'extrait selon l'invention diminuait la quantité de catalase ainsi que la quantité de caspase 3 clivée c'est-à-dire active. Il est connu dans la littérature, que l'activité de la catalase est augmentée lors d'un stress, notamment UV. C'est ainsi qu'elle sert souvent de marqueur de stress et de formation de radicaux libres dans les cellules soumises à des agressions extérieures (S.K. Katiyar et al., Carcinogenesis, vol.22, n°2, p. 287-294, 2001). La caspase-3 quant à elle, appartient à la famille des caspases, famille qui compte 14 membres à l'heure actuelle. Les caspases sont des protéases à cystéine que l'on classe dans la catégorie des protéines pro-apoptotiques. En effet, le rôle des caspases est principalement exécutif, c'est-à-dire qu'elles vont s'attacher à éteindre les voies protectrices et à activer des molécules qui vont participer à la destruction cellulaire. Toutes les caspases ont une structure très conservée comprenant, un pro-domaine N-

Ainsi, le premier objet de la présente invention se rapporte à l'utilisation d'un hydrolysat peptidique de pois en tant qu'agent actif antioxydant solubilisé dans une composition cosmétique , ledit hydrolysat provenant de l'hydrolyse des protéines du pois *Pisum sativum* et comprenant au moins 70% de composés de nature peptidique de taille inférieure à 5 kDa, pour protéger les fibres kératiniques choisies parmi les cheveux, les poils, les cils et les sourcils, des rayonnements UV, ou des stress oxydatifs dus à des traitements physiques ou chimiques choisis parmi les colorations, les défrisages, les permanentes ou les brushings, par une activité antioxydante au niveau des cellules du follicule pileux.

Un autre objet de la présente invention concerne, d'une part, un procédé de traitement cosmétique non thérapeutique destiné à protéger les fibres kératiniques choisies parmi les cheveux, les poils, les cils et les sourcils, des rayonnements UV, ou des stress oxydatifs dus à des traitements physiques ou chimiques choisis parmi les colorations, les défrisages, les permanentes ou les brushings, par une activité antioxydante au niveau des cellules du follicule pileux, caractérisé en ce que l'on applique quotidiennement ou ponctuellement sur les fibres kératiniques à traiter, une composition dans laquelle est solubilisé un hydrolysat provenant de l'hydrolyse des protéines du pois *Pisum sativum* et comprenant au moins 70% de composés de nature peptidique de taille inférieure à 5 kDa.

La présente invention concerne donc l'utilisation d'un hydrolysat peptidique de pois en tant qu'agent actif antioxydant dans une composition cosmétique destinée au soin des cheveux.

On entend par « hydrolysat peptidique », un extrait comprenant un mélange de composés majoritairement représentés par des peptides ou des oligopeptides. Selon l'invention, on utilisera indifféremment les termes « hydrolysat peptidique », « extrait », « extrait solubilisé » ou « agent actif ».

On entend par « cheveux », l'ensemble des fibres kératiniques humaines et plus précisément les cheveux, les sourcils, les cils, les poils de barbe, de moustache, les poils pubiens et les ongles. Plus spécialement, l'invention s'applique aux cheveux et/ou aux cils humains.

La Demanderesse a découvert qu'un extrait peptidique de pois présente une activité certaine sur les follicules pileux des cheveux et, en particulier, avait une action en tant qu'agent protecteur contre plusieurs types d'agressions extérieures grâce à son action antioxydante. En effet, la Demanderesse a démontré à l'aide de tests *in vitro,* que l'extrait selon l'invention diminuait la quantité de catalase ainsi que la quantité de caspase 3 clivée c'est-à-dire active. Il est connu dans la littérature, que l'activité de la catalase est augmentée lors d'un stress, notamment UV. C'est ainsi qu'elle sert souvent de marqueur de stress et de formation de radicaux libres dans les cellules soumises à des agressions extérieures (S.K. Katiyar et al., Carcinogenesis, vol.22, n°2, p. 287-294, 2001). La caspase-3 quant à elle, appartient à la famille des caspases, famille qui compte 14 membres à l'heure actuelle. Les caspases sont des protéases à cystéine que l'on classe dans la catégorie des protéines pro-apoptotiques. En effet, le rôle des caspases est principalement exécutif, c'est-à-dire qu'elles vont s'attacher à éteindre les voies protectrices et à activer des molécules qui vont participer à la destruction cellulaire. Toutes les caspases ont une structure très conservée comprenant, un pro-domaine N-nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques correspondant aux peptides biologiquement actifs selon l'invention, mais en s'assurant toutefois de la présence desdits fragments par des moyens analytiques appropriés.

Plus particulièrement selon l'invention on utilise des graines de pois *Pisum sativum* L. Le terme pois désigne aussi la graine, elle-même riche en protéines (25 %).

Toute méthode d'extraction ou de purification connue de l'homme du métier peut être utilisée afin de préparer l'hydrolysat selon l'invention.

Dans une première étape, les graines sont broyées à l'aide d'un broyeur à plantes. La poudre ainsi obtenue peut ultérieurement être "délipidée" à l'aide d'un solvant organique classique (comme par exemple un alcool, l'hexane ou de l'acétone).

On réalise ensuite l'extraction des protéines suivant le procédé classique (Osborne, 1924) modifié ; le broyat de plante est mis en suspension dans une solution alcaline contenant un produit adsorbant de type polyvinylpolypyrrolidone (PVPP) insoluble (0,01 -20 %) ; en effet il a été observé que les opérations d'hydrolyses et de purifications ultérieures étaient facilitées par ce moyen. En particulier, la concentration en substances de type phénoliques, interagissant avec les protéines, se trouve nettement réduite.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques. On peut alors citer l'utilisation des endoprotéases d'origine végétale (papaïne, bromelaïne, ficine) et de micro-organismes (Aspergillus, Rhizopus, Bacillus, etc.). Pour les mêmes raisons que précédemment, c'est-à-dire l'élimination des substances polyphénoliques, une quantité de polyvinylpolypyrrolidone est additionnée au milieu réactionnel lors de cette étape d'hydrolyse ménagée. Après désactivation thermique des enzymes et filtration, permettant d'éliminer les enzymes résiduelles et les polymères, le filtrat (solution) obtenu est encore purifié afin de sélectionner les composés de nature peptidique de bas poids moléculaires. Le fractionnement peut s'effectuer avantageusement par ultrafiltration et/ ou par une méthode de type chromatographique.

A cette étape, l'hydrolysat de pois est caractérisé par un poids sec de 70 à 80 g/kg, un taux de protéines de 55 à 65 g/l, un taux de sucres de 2 à 5 g/l et un taux de polyphénols de 1 à 3 g/l.

L' hydrolysat obtenu selon l'invention est analysé qualitativement et quantitativement, selon les techniques classiques bien connues de l'homme du métier, pour ses caractéristiques physico-chimiques et sa teneur en composés de nature protéique et peptidique. Ainsi, l'hydrolysat final est composé de peptides de poids moléculaire inférieur à 5 kDa.

On procède ensuite à une phase de dilution dans de l'eau ou dans tout mélange de solvants contenant de l'eau. Ainsi, l'hydrolysat selon l'invention est avantageusement solubilisé dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants. L'agent actif dilué est ensuite stérilisé par filtration stérile.

Après cette étape, l'hydrolysat peptidique de pois est caractérisé par une teneur en composés peptidiques de 0,5 à 5 g/l et préférentiellement au moins entre 2 et 3 g/l. En outre, après analyses qualitatives, il apparaît que l'hydrolysat contient au moins 70 % de composés de nature peptidique de taille inférieure à 5 kDa, et préférentiellement au moins 85 %. Cet hydrolysat peptidique solubilisé correspond à l'agent actif selon l'invention.

Selon un second mode de réalisation de l'invention, l'extrait solubilisé peut être encapsulé ou mis dans un vecteur cosmétique ou pharmaceutique comme les liposomes ou toute autre microcapsule utilisée dans le domaine de la cosmétique, ou adsorbé sur des polymères organiques poudreux, des supports minéraux comme les talcs et bentonites, et plus généralement solubilisé dans, ou fixé sur, tout vecteur physiologiquement acceptable.

Ainsi l'extrait solubilisé est utilisé dans les compositions selon l'invention à une concentration comprise entre 0,001 % à 5 % environ, et préférentiellement à une concentration comprise entre 0,01 % et 1 % environ par rapport au poids total de la composition finale.

Préférentiellement, la composition selon l'invention se présente sous une forme adaptée à l'application par voie topique sur les cheveux.

La composition utilisable selon l'invention peut en particulier consister en une composition pour soins capillaires, et notamment un shampooing, un après-shampooing, une lotion de mise en plis, une lotion traitante pré- ou post- traitement agressif pour les cheveux, une crème ou un gel coiffant, une lotion restructurante pour les cheveux, un masque, de mousse traitante etc.

La composition pourra notamment être sous forme de crème, émulsion huile-dans-eau, eau-dans-huile, ou émulsions multiples de type huile-dans-eau-dans-huile ou eau-dans-huile-dans-eau, suspension, gel aqueux, solutions aqueuses, hydroalcooliques, ou huileuses. La composition peut être plus ou moins fluide et se présenter sous la forme d'une crème blanche ou colorée, d'une pommade, d'un lait, d'une lotion, d'un sérum, d'une mousse, d'une biphasé, ou encore sous forme d'un aérosol.

Enfin, la composition pourra comprendre tout additif communément utilisé dans le domaine d'application envisagé ainsi que les adjuvants nécessaires à leur formulation, tels que des co-solvants (éthanol, glycérol, alcool benzylique, humectant...), des épaississants, des diluants, des émulsionnants, des anti-oxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut par exemple citer des polymères hydrosolubles de type polymère naturel, tels que les polysaccharides, ou polypeptides, des dérivés cellulosiques de type méthylcellulose ou hydroxypropylcellulose, ou encore des polymères synthétiques, polaxamères, carbomères, PVA ou PVP et notamment les polymères vendus par la société ISP.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition selon l'invention est une émulsion, la phase grasse peut représenter de 5 à 80 % en poids, et de préférence de 5 à 50 %, par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids par rapport au poids total de la composition.

Par ailleurs, la composition selon l'invention peut comprendre en outre, au moins un composé améliorant la pousse et/ou la santé des cheveux.

On peut citer notamment des vitamines, d'autres extraits peptidiques végétaux, le minoxidil, des esters de l'acide nicotinique, des oligo-éléments, des agents anti-inflammatoires, de l'acide rétinoïque ou ses dérivés, du rétinol, des inhibiteurs de la 5α-réductase ou des composés peptidiques issus de la synthèse chimique. Comme exemple de vitamine on peut citer les vitamines A, E, B5, B6, C, H, ou PP, comme exemple d'oligo-éléments on peut citer le zinc, le cuivre, le magnésium, ou encore le silicium.

Un autre objet de l'invention concerne un procédé de traitement cosmétique non thérapeutique destiné à protéger les cheveux des agressions extérieures par application quotidienne ou ponctuelle sur la zone du cuir chevelu à traiter, d'une composition comprenant un hydrolysat selon l'invention. Un autre objet concerne un procédé de traitement cosmétique non thérapeutique destiné à protéger les cheveux des stress oxydatifs, par application quotidienne ou ponctuelle d'une composition selon l'invention. Préférentiellement, la composition sera appliquée avant un traitement chimique de type permanente, défrisage, ou encore coloration. Ainsi ladite composition pourra être utilisée en tant que pré-traitement par les professionnels de la coiffure par exemple. Des tests réalisés en *in-vitro* ont permis de démontrer par exemple, qu'en cas de stress oxydatif du à l'H2O2, l'apparition de vacuoles dans les cellules de la couche externe de la gaine externe du follicule pileux était moins importante en cas de pré-traitement par l'actif que sans. Selon un autre mode de réalisation, la composition sera appliquée avant une exposition au soleil afin de prévenir des dommages dus aux rayonnements UV. Des résultats identiques ont été obtenus lors de stress induits par une exposition à des rayonnements UV. Ces tests ont ainsi démontrés l'activité d'antioxydant de l'hydrolysat selon l'invention.

Enfin, un dernier objet de l'invention concerne un procédé de traitement cosmétique non thérapeutique destiné à protéger les cils des agressions extérieures et des stress oxydatifs, par application quotidienne ou ponctuelle sur les cils, d'une composition comprenant un hydrolysat selon l'invention.

D'autres avantages et caractéristiques de l'invention apparaîtront mieux à la lecture des exemples donnés à titre illustratif et non limitatif.

### Exemple 1 : Préparation d'un hydrolysat peptidique enrichi en peptides bioactifs à partir de pois (Pisum sativum L.)

L'hydrolysat peptidique est obtenu à partir d'un extrait de plantes de l'espèce *Pisum sativum L.* Bien entendu l'extrait peut être préparé à partir de plantes d'au moins l'une quelconque des nombreuses variétés et espèces appartenant au genre Pisum.

Dans une première étape, 1 kg de pois décortiqués sont délipidés par l'action d'un solvant organique : l'hexane.

La farine de pois ainsi obtenue est mise en solution dans 10 volumes d'eau en présence de 2 % de POLYCLAR® 10 (polyvinylpolypyrrolidone - PVPP - insoluble). Le mélange est ajusté à un pH compris entre 7 et 8 avec une solution aqueuse de soude 1 M.

Après ajustement du pH, il est rajouté 2 % de flavourzym® dans le milieu réactionnel. L'hydrolyse est obtenue après 2 heures d'agitation à 50°C. On procède à l'inactivation de l'enzyme en chauffant la solution à 80°C pendant 2 heures. Le mélange réactionnel ainsi obtenu correspond à l'extrait de pois.

Le procédé de purification commence par des filtrations successives à l'aide de filtres-plaques Seitz-Orion de porosité décroissante (jusqu'à 0,2 µm) afin d'obtenir une solution brillante et limpide. A cette étape, l'hydrolysat de pois est caractérisé par un sec titrant de 70-80 g/kg, un taux de protéines de 55-65 g/l, un taux de sucres de 2-5 g/l et un taux de polyphénol de 1-3 g/l.

La nature protéique de cet hydrolysat est mise en évidence par électrophorèse sur gel de polyacrylamide. Pour cette analyse, on utilise les gels NuPAGE® Bis-Tris Pre-cast (Invitrogen). L'hydrolysat peptidique de pois est chauffé à 70°C pendant 10 minutes dans des conditions réductrices dénaturantes dans un tampon de préparation d'échantillon NuPAGE® LDS. La migration des protéines est réalisée à l'aide du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe 2 grandes familles de protéines : la 1ère famille correspond à des protéines de poids moléculaire de 20 à 25 kDa et la dernière famille à des protéines de poids moléculaires inférieurs à 5 kDa.

Cette solution est alors purifiée en éliminant les protéines de poids moléculaires supérieurs à 5 kDa à l'aide d'une filtration à flux tangentiel. Pour cela, l'hydrolysat de pois est pompé sous pression à travers un support Pellicon® équipé de cassette Pellicon® 2 Biomax 30 kDa. Ce 1er filtrat est récupéré pour être ensuite filtré à travers une autre cassette Pellicon® 2 Biomax 5 kDa. En fin de purification, on obtient un hydrolysat peptidique de pois jaune-beige, brillant et limpide. Il est caractérisé par un sec de 50 à 55 g/kg, une teneur en protéines de 50 à 52 g/l.

L'extrait obtenu selon le procédé décrit dans l'exemple 1 est ensuite solubilisé dans du glycérol.

### Exemple 2 : Action de l'hydrolysat peptidique de pois sur des biopsies de cuir chevelu maintenues en culture in vitro : immunomarquages de la catalase, de p63 et de la caspase-3

### 2.1 Cultures de biopsies de cuir chevelu et inclusion des coupes

Des biopsies de peaux (issues de liftings) présentant des cheveux sont cultivées de la même manière que des explants de peaux. Des biopsies de 6 mm sont réalisées au moyen de punch de biopsies et mises en culture sur des inserts dans un milieu WILLIAM E. en présence de Primocine (Invivogen), 10 µg/ml d'insuline 10 nG/ml, d'Hydrocortisone et 2 mmol/L de L-Glutamine.

Les explants de peaux sont déposés dans des plaques 6 puits et traités ou non en mettant 20 µl d'extrait à 1 % dilué dans du PBS, en contact sur les biopsies, pendant 24 heures de culture. A la fin de l'expérience, l'enrobage des explants peut se faire soit dans la paraffine, soit dans de l'OCT suivi d'une congélation. Dans le cas de l'enrobage en paraffine, les biopsies sont mises en cassette et plongées dans un mélange de formol à 10 % pendant 2 heures dans un appareil automatisé (VIP). Le type d'enrobage choisi dépendra du marqueur à tester (catalase, soit p-63, soit caspase-3). Par exemple, dans le cas d'un marquage de la catalase, l'enrobage sera fait dans de la paraffine. Pour cela, les biopsies sont enrobées à l'aide de paraffine, suivie par une série de bains d'alcool (à concentration et temps croissants), suivie de 2 bains de xylène et enfin d'un bain de paraffine. La durée totale de cette série d'opérations est d'une douzaine d'heures. Les biopsies ainsi enrobées sont ensuite placées dans des cassettes appropriées, orientées et mises dans un bloc de paraffine afin d'être ensuite coupées à 4 µm par un microtome. Les coupes paraffinées sont ensuite déparaffinées et réhydratées avant la mise de l'anticorps. Pour cela, une série de bains de xylène, suivie d'une série de bains d'alcool (à concentration et temps croissants) et d'un rinçage dans de l'eau puis dans du PBS sont réalisées à cet effet.

### 2.2 Immunomarquage de catalase (protocole paraffine)

Les coupes déparaffinées sont rincées au PBS pendant 2 minutes, puis entourées, et on incube chaque coupe dans 100 µl de BSA 5 % pendant 30 minutes. Puis, 100 µl d'anticorps primaire anti-catalase dilué au 1/100^{ième} (Calbiochem, Rabbit polyclonal) sont ajoutés et mis pendant 60 minutes sous agitation en chambre humide. Après rinçage avec du PBS pendant 30 minutes, 100 µL d'anticorps secondaire dilué au 1/1000^{ième} (Invitrogene, Alexa Fluoro 488 anti-Rabbit) marqué à la fluorescence sont ajoutés et laissés durant 1 heure à l'obscurité sous agitation en chambre humide. Ces marquages sont réalisés en parallèle sur des biopsies pré-traitées ou non pendant 24 heures avec l'extrait peptidique actif à 1 %, et en présence ou non d'un stress UV (5 J/cm² + 200 mJ/cm²). Les lames sont ensuite rincées dans le PBS, montées entre lame et lamelle dans l'Aquatex et l'observation s'effectue au microscope à épifluorescence.

### Résultats

En condition contrôle sans UV, on constate une quantité plus importante de catalase dans les biopsies non traitées avec l'actif. Cette quantité de catalase est encore plus importante en condition avec UV dans les biopsies non prétraitées. Même si l'on constate une augmentation de la quantité de catalase en condition prétraitée, cette quantité est très inférieure à celle obtenue dans les biopsies non prétraitées et avec UV. Par conséquent, on peut conclure que l'actif selon l'invention a un effet de protection des cellules composant le follicule pileux.

Le même protocole a été répété mais cette fois-ci avec un stress oxydatif provoqué par l'adjonction de péroxyde d'hydrogène (H2O2). On constate alors là aussi, une quantité plus importante de catalase dans les biopsies n'ayant pas reçu un prétraitement à l'aide de l'actif selon l'invention. On constate ainsi là encore, un rôle protecteur de l'extrait peptidique de pois sur les cellules du follicule pileux.

### 2.3 Immunomarquage de p63 (protocole avec l'OCT)

Afin de réaliser un marquage de p63 dans les follicules pileux, des biopsies de lifting sont traitées comme décrits plus haut paragraphe 2.1. Le marquage est réalisé en condition contrôle avec ou sans prétraitement des biopsies à l'aide de l'actif à 1 %, et avec ou sans un stress par rayonnements UV (5 J/cm² + 200 mJ/cm²). Pour cela, les explants de peaux sont déposés dans des plaques 6 puits et les follicules sont traités ou non en mettant 20 µl d'extrait actif à 1 %, en contact sur les biopsies, après 24 heures de culture. Par la suite, afin de préparer le marquage par anticorps, les coupes ne sont pas paraffinées mais mises dans l'OCT (optimum cutting température) et refroidies rapidement dans un bain d'azote liquide, puis coupées au cryostat (frozen section). Les biopsies sont donc prises et inclues dans l'OCT qui se solidifie au froid (les blocs sont conservées à -20°C) ; les blocs ainsi solidifiés sont ensuite coupés au cryotome où des coupes de 6 µm sont réalisées. Les coupes sont recueillies sur des lames d'observation poly-lysinées. Les coupes sont ensuite mises à température ambiante avant d'être utilisées pour l'immunomarquage par ajout de 100 µl d'un anticorps primaire anti-p63 (Santa Cruz, mouse monoclonal) dilué au 1/100^{ième}. Après mise sous agitation pendant 60 minutes, 100 µl d'un anticorps secondaire dilué au 1/1000^{ième} (Invitrogene, Alexa Fluoro 488 anti-Rabbit) marqué à la fluorescence, sont ajoutés et laissés durant 1 heure à l'obscurité sous agitation en chambre humide. Les lames sont ensuite rincées dans le PBS, montées entre lame et lamelle et l'observation s'effectue au microscope à épifluorescence.

### Résultats

On constate en condition contrôle, que le marquage p63 diminue lors du stress UV. Par contre, lorsque l'actif est ajouté en prétraitement, le marquage de p63 est certes diminué par rapport à la condition contrôle (sans UV avec actif), mais cette baisse est atténuée par rapport à la condition contrôle avec UV et sans actif. Par conséquent, l'hydrolysat peptidique selon l'invention a donc joué un rôle protecteur au niveau des cellules du follicule pileux par limitation de la baisse de protéine p63.

Un protocole identique a été mis en place, dans les mêmes conditions expérimentales, afin de tester l'effet protecteur de l'actif sur des biopsies soumises à un stress oxydatif par l'H2O2. On constate alors l'effet antioxydant de l'hydrolysat peptidique de pois sur les biopsies en présence et même en absence de stress oxydatif.

### 2.4 Immunomarquage de la caspase-3 (protocole paraffine avec démasquage au micro-onde)

Les coupes déparaffinées sont rincées au PBS pendant 2 minutes, puis entourées, et on incube chaque coupe dans 100 µl de BSA à 5 % pendant 30 minutes. Puis, 100 jj.1 d'anticorps primaire anti-caspase-3 clivée dilué au 1/200^{ième} (Cell Signaling, rabbit polyclonal) sont ajoutés et mis pendant 60 minutes sous agitation en chambre humide. Après rinçage avec du PBS pendant 30 minutes, 100 µL d'anticorps secondaire dilué au 1/1000^{ième} (Invitrogene, Alexa Fluoro 488 anti-Rabbit) marqué à la fluorescence sont ajoutés et laissés durant 1 heure à l'obscurité sous agitation en chambre humide. Ces marquages sont réalisés en parallèle sur des biopsies pré-traitées ou non pendant 24 heures avec l'extrait peptidique actif à 1 %, et en présence ou non d'un stress UV (5 J/cm² + 200 mJ/cm²). Les lames sont ensuite rincées dans le PBS, montées entre lame et lamelle dans l'Aquatex et l'observation s'effectue au microscope à épifluorescence.

### Résultats

Dans la condition contrôle sans UV, on constate une quantité plus importante de caspase-3 clivée (et donc active) dans les coupes non prétraitées par rapport aux coupes prétraitées. Lors de l'application du stress UV, on constate une augmentation du marquage p63 dans les cellules prétraitées et non prétraitées. Cependant, le marquage est beaucoup moins important dans les biopsies prétraitées par rapport aux biopsies non traitées. On peut donc conclure que le prétraitement à l'aide de l'actif selon l'invention a permis de limiter la formation de caspase-3 clivée et active.

Le même protocole a été répété mais avec cette fois-ci un stress provoqué par l'application d'une solution d' H2O2 au lieu des rayonnements UV. Là encore on constate qu'en présence du stress d' H2O2, le marquage caspase-3 activée est plus important dans les coupes non prétraitées que dans les coupes prétraitées.

Par conséquent, l'hydrolysat selon l'invention a joué un rôle d'antioxydant en limitant la quantité de caspase activée.

### Exemple 3 : Action de l'hydrolysat peptidique de pois sur des follicules maintenus en culture in vitro : Coloration Hematoxyline/Eosine(H/E)

Des biopsies de peaux (issues de liftings) présentant des cheveux sont cultivées de la même manière que des explants de peaux. Des biopsies de 6 mm sont réalisées au moyen de punch de biopsies et mises en culture sur des inserts dans un milieu WILLIAM E. en présence d'insuline 10 nG/ml, d'Hydrocortisone et 2 mmol/L de L-Glutamine.

Les explants de peaux sont déposés dans des plaques 6 puits et les follicules sont prétraités en mettant 20 µl d'extrait à 1 % en contact sur les biopsies pendant 24 heures. Les biopsies sont ensuite :
- dans la condition 1, soumises à une irradiation UVA à 5 J/cm², suivie d'une irradiation UVB à 200 mJ/cm²,
- dans la condition 2, soumises à un stress par application d'une solution d'H2O2 à 5 mM.

Les biopsies sont ensuite remises pendant 24 heures supplémentaires. Des boîtes contrôle avec des explants non prétraités par l'extrait peptidique actif mais irradiés servent de témoin. Une évaluation de l'état des cellules après irradiation UV est réalisée par un marquage hématoxyline/éosine.

### Résultats

La forme générale de l'outer root sheet (ORS) est irrégulière dans les follicules qui n'ont pas été prétraités par l'extrait peptidique, alors que celle-ci est tout à fait régulière dans les follicules prétraités. En outre, les cellules des follicules pileux non prétraités présentent des dommages dus aux rayonnements UV, dommages tels que l'apparition de vacuoles, d'oedèmes et de cellules apoptotiques. Toutes ces manifestations ne sont pas observées dans les follicules prétraités par l'extrait peptidique. On en déduit donc un rôle protecteur de cet extrait peptidique de pois sur les follicules pileux et, entre autre, sur les cellules de la couche externe de la gaine externe du follicule.

### Exemple 4 : Préparation de compositions

### 1. Sérum hydratant le cuir chevelu et les cheveux

Disperser le Lubragel NP et le Lubragel IIXD dans l'eau sous agitation. Ajouter l'AMP 95 et mélanger jusqu'à dissolution. Ajouter le Styleze® 2000, et agiter jusqu'à dissolution et obtention d'un aspect uniforme. Laisser refroidir à température ambiante et ajouter les ingrédients restants dans l'ordre de la liste en agitant jusqu'à obtenir un aspect uniforme entre chacun d'eux.

| **Ingrédients (INCI/Trade name)** | **%W/W** | **Supplier** |
|---|---|---|
| Eau desionisée | 71.37 | |
| Propanol Aminométhyle (AMP 95) | 0.13 | |
| Styleze 2000 | 0.50 | |
| SiTec DMC 6031 | 1.00 | |
| Polyméthacrylate Glycéryl/Lubrajel® NP | 24.50 | ISP |
| Glycérine et Polyacrylate Glycéryl/ Lubrajel® IIXD | 1.00 | ISP |
| Propylène Glycol Urée Diazolidinyle Iodopropynyl Butylcarbamate | 0.50 | ISP |
| *Extrait de pois selon l'invention* | 1.00 | ISP |
| Total | 100.00 | |

Appliquer le produit sur le cuir chevelu humide. Masser pour répartir uniformément le produit. Le sérum favorise la pousse et/ou la repousse des cheveux tout en les rendant plus nerveux.

### 2. Lait/ spray conditionner

Mettre l'eau dans un récipient convenable et commencer l'agitation. Ajouter le Gafquat 755N et le Liquid Germall Plus et agiter jusqu'à obtenir un aspect uniforme. Ajouter le RapiThix A-60 et agiter jusqu'à obtenir un aspect uniforme (environ 15 minutes). Ajouter l'hydrolysat selon l'invention et agiter jusqu'à obtenir un aspect uniforme. Disposer le produit dans un vaporisateur non-aérosol équipé d'une pompe Calmar pump Mark VI WL31.

| **Ingrédients (INCI/Trade name)** | **%W/W** | **Supplier** |
|---|---|---|
| Eau dé-ionisée | 92.68 | |
| Polyquaternium-11/Gafquat® 755N | 3.50 | ISP |
| PEG/PPG-25/25 Dimethicone /SiTec DMC 6031 | 1.00 | ISP |
| Propylène Glycol | 0.50 | ISP |
| Urée Diazolidinyl | | |
| Iodopropynyl Butylcarbamate/ | | |
| Sodium Polyacrylate Polydecene Hydrogéné Trideceth-6/ | 0.32 | ISP |
| *Polectron 430* | 1.00 | ISP |
| *Extrait de pois selon l'invention* | 1.00 | ISP |
| Total | 100.00 | |

Le produit est conçu pour être vaporisé sur le cuir chevelu et sur cheveux humides. Masser pour répartir uniformément le produit. Le lait ainsi proposé permet de protéger les cheveux tout en les rendant lisses et faciles à coiffer.

## Revendications

1. Utilisation d'un hydrolysat peptidique de pois en tant qu'agent antioxydant dans une composition cosmétique, ledit hydrolysat provenant de l'hydrolyse des protéines du pois *Pisum sativum* et comprenant au moins 70% de composés de nature peptidique de taille inférieure à 5 kDa, pour protéger les fibres kératiniques choisies parmi les cheveux, les poils, les cils et les sourcils, des rayonnements UV ou des stress oxydatifs dus à des traitements physiques ou chimiques choisis parmi les colorations, les défrisages, les permanentes ou les brushings, par une activité antioxydante au niveau des cellules du follicule pileux.

2. Utilisation selon la revendication 1, **caractérisée en ce que** l'hydrolysat comprend au moins 85% de composés de nature peptidique.

3. Utilisation selon l'une des revendications 1 ou 2, **caractérisée en ce que** l'hydrolysat est solubilisé dans un ou plusieurs solvants physiologiquement acceptables choisis parmi l'eau, le glycérol, l'éthanol, le propylène glycol, le butylène glycol, le dipropylène glycol, les diglycols éthoxylés ou propoxylés, les polyols cycliques ou tout mélange de ces solvants.

4. Utilisation selon l'une quelconque des revendications précédentes, **caractérisée en ce que** l'hydrolysat solubilisé comprend au moins entre 0,5 et 5 g/l de composés de nature peptidique.

5. Utilisation selon la revendication 4, **caractérisée en ce que** l'hydrolysat solubilisé comprend au moins entre 2 et 3 g/l de composés de nature peptidique.

6. Utilisation selon l'une quelconque des revendications précédentes, **caractérisée en ce que** l'hydrolysat solubilisé est utilisé en une quantité représentant de 0,001 % à 5 % du poids total de la composition.

7. Utilisation selon la revendication 6, **caractérisée en ce que** l'hydrolysat solubilisé est utilisé en une quantité représentant de 0,01 % à 1 % du poids total de la composition.

8. Utilisation selon l'une quelconque des revendications précédentes, **caractérisée en ce que** la composition comprend en outre, au moins un composé améliorant la pousse et/ou la santé des cheveux.

9. Utilisation selon la revendication 8, **caractérisée** en ce ledit composé est choisi parmi des vitamines, d'autres extraits peptidiques végétaux, le minoxidil, des esters de l'acide nicotinique, des oligo-éléments, des agents anti-inflammatoires, de l'acide rétinoïque ou ses dérivés, du rétinol, des inhibiteurs de la 5a-réductase ou des composés peptidiques issus de la synthèse chimique.

10. Procédé de traitement cosmétique non thérapeutique destiné à protéger les fibres kératiniques choisies parmi les cheveux, les poils, les cils et les sourcils, des rayonnements UV ou des stress oxydatifs dus à des traitements physiques ou chimiques choisis parmi les colorations, les défrisages, les permanentes ou les brushings, par une activité antioxydante au niveau des cellules du follicule pileux, **caractérisé en ce que** l'on applique quotidiennement ou ponctuellement sur les fibres kératiniques à traiter, une composition dans laquelle est solubilisé un hydrolysat provenant de l'hydrolyse des protéines du pois *Pisum sativum* et comprenant au moins 70% de composés de nature peptidique de taille inférieure à 5 kDa.

11. Procédé selon la revendication 10, **caractérisé en ce que** la composition est appliquée avant un traitement chimique des cheveux choisi parmi les permanentes, les défrisages, ou encore les colorations.

12. Procédé selon la revendication 10, **caractérisé en ce que** la composition est appliquée avant une exposition au soleil afin de prévenir des dommages dus aux rayonnements UV.

## Patentansprüche

1. Verwendung eines Peptidhydrolysats aus Erbsen als Antioxidationsmittel in einer kosmetischen Zusammensetzung, wobei das Hydrolysat aus der Hydrolyse der Proteine der Erbse *Pisum sativum* stammt und mindestens 70 % Verbindungen peptidischer Art mit einer Größe von weniger als 5 kDa umfasst, um die keratinischen Fasern, ausgewählt aus den Haaren, den Körperhaaren, den Wimpern und den Augenbrauen, vor den UV-Strahlen oder oxidative Stresssituationen aufgrund physischer oder chemischer Behandlungen, ausgewählt aus den Färbungen, den Glättungen, den Dauerwellen oder den Fönwellen, durch eine antioxidante Aktivität auf der Ebene der Zellen der Haarfollikel zu schützen.

2. Verwendung nach Anspruch 1, **dadurch gekennzeichnet, dass** das Hydrolysat mindestens 85 % Verbindungen peptidischer Art umfasst.

3. Verwendung nach einem der Ansprüche 1 oder 2, **dadurch gekennzeichnet, dass** das Hydrolysat in einem oder in mehreren physiologisch annehmbaren Lösemitteln solubilisiert ist, ausgewählt aus Wasser, Glycerol, Ethanol, Propylenglycol, Butylenglycol, Dipropylenglycol, ethoxylierten oder propoxylierten Diglykolen, cyclischen Polyolen oder jeder Mischung dieser Lösemittel.

4. Verwendung nach einem der vorhergehenden Ansprüche, **dadurch gekennzeichnet, dass** das solubilisierte Hydrolysat mindestens zwischen 0,5 und 5 g/l Verbindungen peptidischer Art umfasst.

5. Verwendung nach Anspruch 4, **dadurch gekennzeichnet, dass** das solubilisierte Hydrolysat mindestens zwischen 2 und 3 g/l Verbindungen peptidischer Art umfasst.

6. Verwendung nach einem der vorhergehenden Ansprüche, **dadurch gekennzeichnet, dass** das solubilisierte Hydrolysat in einer Menge verwendet wird, die 0,001 % bis 5 % des Gesamtgewichts der Zusammensetzung darstellt.

7. Verwendung nach Anspruch 6, **dadurch gekennzeichnet, dass** das solubilisierte Hydrolysat in einer Menge verwendet wird, die 0,01 % bis 1 % des Gesamtgewichts der Zusammensetzung darstellt.

8. Verwendung nach einem der vorhergehenden Ansprüche, **dadurch gekennzeichnet, dass** die Zusammensetzung außerdem mindestens eine Verbindung umfasst, die das Wachstum und/oder die Gesundheit der Haare verbessert.

9. Verwendung nach Anspruch 8, **dadurch gekennzeichnet, dass** die Verbindung ausgewählt ist aus Vitaminen, anderen pflanzlichen peptidischen Extrakten, Minoxidil, Estern der Nikotinsäure, Oligoelementen, entzündungshemmenden Mitteln, Retinsäure oder ihren Derivaten, Retinol, Inhibitoren der 5α-Reduktase oder peptidischen Zusammensetzungen, die aus der chemischen Synthese stammen.

10. Verfahren zur kosmetischen, nicht therapeutischen Behandlung, das ausgelegt ist, um die keratinischen Fasern, ausgewählt aus den Haaren, den Körperhaaren, den Wimpern und den Augenbrauen, vor den UV-Strahlen oder oxidativen Stresssituationen aufgrund physischer oder chemischer Behandlungen, ausgewählt aus den Färbungen, den Glättungen, den Dauerwellen oder den Fönwellen durch eine antioxidative Aktivität auf der Ebene der Zellen der Haarfollikel zu schützen, **dadurch gekennzeichnet, dass** täglich oder punktuell auf die zu behandelnden keratinischen Fasern eine Zusammensetzung aufgebracht wird, in der ein Hydrolysat solubilisiert ist, das aus der Hydrolyse der Proteine der Erbse *Pisum sativum* stammt und mindestens 70 % Verbindungen peptidischer Art mit einer Größe von weniger als 5 kDa umfasst.

11. Verfahren nach Anspruch 10, **dadurch gekennzeichnet, dass** die Zusammensetzung vor einer chemischen Behandlung der Haare, ausgewählt aus den Dauerwellen, den Glättungen oder auch den Färbungen, aufgebracht wird.

12. Verfahren nach Anspruch 10, **dadurch gekennzeichnet, dass** die Zusammensetzung vor einer Aussetzung an die Sonne aufgebracht wird, um Beschädigungen aufgrund UV-Strahlen vorzubeugen.

## Claims

1. Use of a pea peptide hydrolysate as an antioxidant agent in a cosmetic composition, said hydrolysate resulting from the hydrolysis of *Pisum sativum* pea proteins and comprising at least 70% peptide type compounds less than 5 kDa in size, for protecting keratin fibres chosen from among hair, body hair, eyelashes and eyebrows, from UV rays or oxidative stress caused by physical or chemical treatments chosen from among colouring treatments, straightening treatments, perms or blow-dries, with an antioxidant activity at hair follicle cell level.

2. Use according to claim 1, **characterised in that** the hydrolysate comprises at least 85% peptide type compounds.

3. Use according to one of claims 1 or 2, **characterised in that** the hydrolysate is solubilised in one or a plurality of physiologically acceptable solvents chosen from among water, glycerol, ethanol, propylene glycol, butylene glycol, dipropylene glycol, ethoxylated or propoxylated diglycols, cyclic polyols or any mixture of these solvents.

4. Use according to any one of the preceding claims, **characterised in that** the solubilised hydrolysate comprises at least between 0.5 and 5 g/l of peptide type compounds.

5. Use according to claim 4, **characterised in that** the solubilised hydrolysate comprises at least between 2 and 3 g/l of peptide type compounds.

6. Use according to any one of the preceding claims, **characterised in that** the solubilised hydrolysate is used in a quantity representing from 0.001% to 5% of the total weight of the composition.

7. Use according to claim 6, **characterised in that** the solubilised hydrolysate is used in a quantity representing from 0.01% to 1% of the total weight of the composition.

8. Use according to any one of the preceding claims, **characterised in that** the composition further comprises at least one compound enhancing hair growth and/or health.

9. Use according to claim 8, **characterised in that** said compound is chosen from among vitamins, further plant-based peptide extracts, minoxidil, nicotinic acid esters, trace elements, anti-inflammatory agents, retinoic acid or derivatives thereof, retinol, 5α-reductase inhibitors or peptide compounds obtained from chemical synthesis.

10. Non-therapeutic cosmetic treatment method for protecting keratin fibres chosen from among hair, body hair, eyelashes and eyebrows, from UV rays or oxidative stress caused by physical or chemical treatments chosen from among colouring treatments, straightening treatments, perms or blow-dries, with an antioxidant activity at hair follicle cell level, **characterised in that**, daily or occasionally on the keratin fibres to be treated, a composition is applied wherein a hydrolysate resulting from the hydrolysis of *Pisum sativum* pea proteins and comprising at least 70% peptide type compounds less than 5 kDa in size is solubilised.

11. Method according to claim 10, **characterised in that** the composition is applied prior to a chemical hair treatment chosen from among perms, straightening treatments, or colouring treatments.

12. Method according to claim 10, **characterised in that** the composition is applied prior to sun exposure so as to prevent damaged caused by UV rays.
